# EUROPEAN PATENT APPLICATION

(11) **EP 2 404 559 A1**
(43) Date of publication of application: **11.01.2012**
(21) Application number: 11184252.2
(22) Date of filing: 02.02.2006
(51) Int. Cl.: A61B 17/12, A61L 27/38

(54) **Vaso-occlusive devices including non-biodegradable biomaterials**

(30) Priority: 04.02.2005 US 51599
(62) Divisional of application: 06720181.4
(71) Applicant: Stryker Corporation, Kalamazoo, MI 49002 (US); Stryker NV Operations Ltd, Dublin 2 (IE)
(72) Inventor: Carr-Brendel, Victoria, E., Pleasanton, CA California 94566 (US)
(74) Representative: Loichen, Martin

(57) **Abstract**

This is a device for occluding a space within the body. In particular, the device comprises a plurality of strands (10) comprising a porous, non-biodegradable material having a pore size of less than about 20 microns, and a vaso-occlusive inner member, wherein the non-biodegradable material is attached to the inner member at one or more locations.

## Description

### FIELD OF THE INVENTION

Compositions and methods for repair of aneurysms are described. In particular, vaso-occlusive devices are disclosed, as are methods of making and using these devices.

### BACKGROUND

An aneurysm is a dilation of a blood vessel that poses a risk to health from the potential for rupture, clotting, or dissecting. Rupture of an aneurysm in the brain causes stroke, and rupture of an aneurysm in the abdomen causes shock. Cerebral aneurysms are usually detected in patients as the result of a seizure or hemorrhage and can result in significant morbidity or mortality.

There are a variety of materials and devices which have been used for treatment of aneurysms, including platinum and stainless steel microcoils, polyvinyl alcohol sponges (Ivalone), and other mechanical devices. For example, vaso-occlusion devices are surgical implements or implants that are placed within the vasculature of the human body, typically via a catheter, either to block the flow of blood through a vessel making up that portion of the vasculature through the formation of an embolus or to form such an embolus within an aneurysm stemming from the vessel. One widely used vaso-occlusive device is a helical wire coil having windings which may be dimensioned to engage the walls of the vessels. (*See, e.g.,* U.S. Patent No. 4,994,069 to Ritchart et al.) Other less stiff helically coiled devices have been described, as well as those involving woven braids. *See, e.g.,* U.S. Patent No. 6,299,627.

U.S. Pat. No. 5,354,295 and its parent, U.S. Pat. No. 5,122,136, both to Guglielmi et al., describe an electrolytically detachable embolic device. Vaso-occlusive coils having little or no inherent secondary shape have also been described. For instance, co-owned U.S. Patent Numbers 5,690,666; 5,826,587; and 6,458,119 by Berenstein et al., describes coils having little or no shape after introduction into the vascular space. U.S. Patent No. 5,382,259 describes non-expanding braids covering a primary coil structure.

Vaso-occlusive devices comprising one or more coatings have also been described. U.S. Patent Nos. 6,280,457 discloses vaso-occlusive devices that include biodegradable coatings.

However, none of the above documents show a device as described herein including one or more non-biodegradable materials that limit the formation of scar tissue and resist long-term recanalization of an aneurysm.

### SUMMARY OF THE INVENTION

Thus, this invention includes novel occlusive compositions as well as methods of using and making these compositions.

In one aspect, described herein is a vaso-occlusive device comprising a porous, non-biodegradable material having a pore size of less than about 20 microns. In certain embodiments, the non-biodegradable material comprises a polymer (*e.g*, cellulose acetate, mixed esters cellulose, PTFE/polyester, acrylic copolymers or combinations thereof. The devices described herein may have a linear primary configuration prior to deployment and a secondary three-dimensional configuration after deployment.

In other aspects, any of the devices described herein may further comprise an inner member. In certain embodiments, the non-biodegradable material is attached to the inner member at one or more locations. In any of the devices described herein, the inner member may comprise a metal (*e.g*., nickel, titanium, platinum, gold, tungsten, iridium and alloys or combinations thereof), stainless steel or a super-elastic metal alloy. The inner member may be, for example, be shaped as a helical coil. In any of the devices described herein, the inner member may further comprise a biodegradable material and/or a bioactive component.

Any of the devices described herein may further comprise a severable junction detachably which may be connected to a pusher element. The detachment junction can be positioned anywhere on the device, for example at one or both ends of the device. In certain embodiments, the severable junction(s) are, an electrolytically detachable assembly adapted to detach by imposition of a current; a mechanically detachable assembly adapted to detach by movement or pressure; a thermally detachable assembly adapted to detach by localized delivery of heat to the junction; a radiation detachable assembly adapted to detach by delivery of electromagnetic radiation to the junction or combinations thereof. The detachment junction(s) may be attached to inner member or non-biodegradable material.

In another aspect, a method of occluding a body cavity is described, the method comprising introducing a vaso-occlusive device as described herein into the body cavity. In certain embodiments, the body cavity is an aneurysm.

These and other embodiments of the subject invention will readily occur to those of skill in the art in light of the disclosure herein.

The invention may also be directed to the following subject-matter:
A vaso-occlusive device comprising a porous, non-biodegradable material having a pore size of less than about 20 microns.

The non-biodegradable material may comprise a polymer.

Preferably, the polymer is selected from the group consisting of cellulose acetate, mixed esters cellulose, PTFE/polyester, acrylic copolymers and combinations thereof.

The device may have a linear primary configuration prior to deployment and a secondary three-dimensional configuration after deployment.

The device may further comprise an inner member, wherein the non-biodegradable material may be preferably attached to the inner member at one or more locations.

The inner member may comprise a metal which may preferably be selected from the group consisting of nickel, titanium, platinum, gold, tungsten, iridium and alloys or combinations thereof. More preferably, the metal may be nitinol or platinum.

According to another aspect, the inner member may comprise a coil. The coil may comprise a stainless steel or super-elastic metal alloy.

The inner member may also comprise a biodegradable material and/or a detachment junction. The detachment junction preferably comprises an electrolytically detachable end adapted to detach from a pusher by imposition of a current on the pusher.

Moreover, the device may further comprise an additional component which is preferably bioactive.

The invention may also be directed to a method of occluding a body cavity comprising introducing the above vaso-occlusive device. In particular, the body cavity may be an aneurysm.

### BRIEF DESCRIPTION OF THE FIGURES

FIG. 1 is a perspective view of an exemplary vaso-occlusive device as described herein made up of strands comprising non-degradable biomaterials.
FIG. 2 is a perspective view of an exemplary vaso-occlusive device as described herein made up of strands, comprising non-degradable biomaterials in combination with a coil-shaped inner member. Also shown is a detachment junction on the inner coil member.
FIG. 3 is a perspective view of an exemplary vaso-occlusive device as described herein as deployed within an aneurysm. The device assumes a random, three- dimensional configuration upon deployment.
FIG. 4 is a reproduction of an electron micrograph showing the pore structure of an exemplary non-degradable biomaterial attached to an inner platinum coil.

It is to be understood that the drawings depict only exemplary embodiments and are not to be considered limiting in scope.

### DESCRIPTION OF THE INVENTION

Occlusive (e.g., embolic) compositions are described. The compositions described herein find use in vascular and neurovascular indications and are particularly useful in treating aneurysms, for example small-diameter, curved or otherwise difficult to access vasculature, for example aneurysms, such as cerebral aneurysms. Methods of making and using these vaso-occlusive elements also form aspects of this invention.

All documents (publications, patents and patent applications) cited herein, whether above or below, are hereby incorporated by reference in their entireties.

It must be noted that, as used in this specification and the appended claims, the singular forms "a", "an", and "the" include plural referents unless the content clearly dictates otherwise. Thus, for example, reference to a device comprising "a strand" includes devices comprising of two or more elements.

The devices described herein comprise a non-biodegradable, typically porous component that aids arrest the foreign body response when implanted into a subject. By "non-biodegradable" or "non-degradable" is meant that a material that persists in the subject in essentially the same form as implanted for a period of time ranging from months to years. Similarly, by "biomaterial" is meant any substance or combination of substances synthetic or natural in origin, which can be used for any period of time, as a whole or as a part of a system that treats, augments, or replaces any tissue, organ, or function in a subject (*e.g.*, mammal).

Aneurysms treated with biodegradable products that promote wound healing may result in the formation of scar tissue over time. Because scar tissue is an avascular, acellular mass made up mostly of extracellular matrix proteins, the interface between the scar tissue and healthy tissue is less robust, than surrounding tissue and, as such, less resistant to long-term recanalization. The vaso-occlusive devices described herein comprise materials that are capable of arresting wound healing in a state of a modified foreign body response (also referred to as material microarchitecture-driven neovascularization) so that the resulting tissue response does not evolve into scar and, as such, is adjacent (continuous) with the healthy tissue and is more resistant to sheer, flow and recanalization. Notably, there is no demarcation between the response to the foreign body and the native tissue -- they are co-continuous.

During a typical course of wound healing, neutrophils are the predominant cell type at the site of injury within the first 24-48 hours killing and phagocytosing bacteria and/or cellular debris. After approximately 48 hours, macrophages become the predominant cell type, further removing cellular and foreign debris from the wounded area. Within three to four days, fibroblasts migrate out of the surrounding connective tissue (*e.g.,* intima) into the wound area and begin to synthesize collagen, which quickly fills the wound space, forming a complex tertiary structure consisting of both cells and extracellular matrix components. New blood vessels also begin to grow into the area at this time to supply oxygen and nutrients needed by the metabolically active fibroblasts and macrophages. However, in a typical course of wound healing, new vessel formation begins to regress in the second week, resulting formation of an avascular and acellular scar.

In contrast to a typical course of wound healing, in a modified foreign body response, material microarchitecture-driven neovascularization of new vessels does not regress and a multitude of vessels persist at the interface of the material and tissue. *See, e.g.,* Padera, et al. (1996) "Time Course of Membrane Microarchitecture-driven Neovascularization," 17 Biomaterials 277-84. The persistence of adjacent vascular structures maintains the integrity of the space filling in-growth with the adjacent preexisting vessel structures in a co-continuous fashion. Furthermore, the in-growth is then available as a substratum upon which endothelium can grow and persist

Thus, the non-biodegradable vaso-occlusive devices as described herein preferably comprise one or more materials that favor an arrested foreign body response, which as described above is granular in nature, has new vessel formation and is typically intimately associated with the non-degradable material. Typically, the non-degradable component comprises one or more materia (*e.g*., polymers) having an average nominal pore size ranging from approximately 0.1 microns to about 20 microns (or any value therebetween), more preferably from about 5 microns to about 15 microns (or any value therebetween), and even more preferably from 0.8 microns to about 10 microns (or any value therebetween), using conventional methods for determination of pore size in the trade.

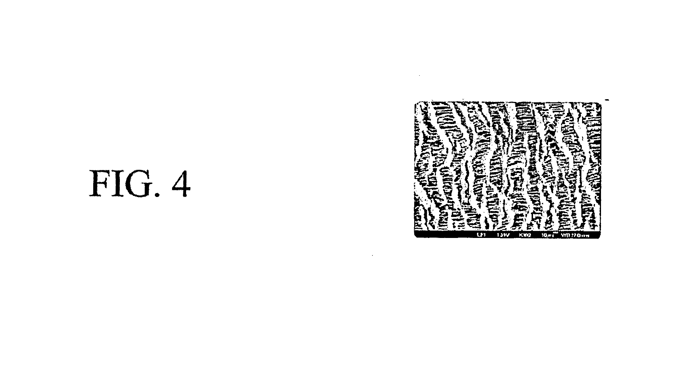

Non-limiting examples of suitable non-degradable materials include, cellulose acetate (pore size range from about 0.8 to about 8 microns); mixed esters cellulose (pore size range from about 1.2 microns to about 8 microns); PTFE/polyester (pore size range from about 1.0 microns to about 10 microns, see, also FIG. 4); and acrylic copolymer (pore size range from about 0.8 microns to about 10 microns). Although the use of some of these materials as chambers for living cells *(see, e.g.,* U.S. Patent Nos. 6,773,458; 5,964,804; 5,807,406) and as vascular grafts (*see, e.g*., U.S. Patent No. 6,517,571) has been described, they have not been used as vaso-occlusive devices, likely because of their known antithrombogenic characteristics.

The non-degradable components of the devices may take a variety of three-dimensional configurations upon deployment into the subject, including structural elements which provide the three dimensional conformation may include fibers, strands, coils, globules, cones or rods of amorphous or uniform geometry which are smooth or rough. Generally, the three-dimensional configuration of the non-degradable component(s) have one dimension larger than the other two and the smaller dimensions preferably do not exceed five microns.

The non-degradable component can also be optionally used in combination with other vaso-occlusive devices, for example the GDC-type vaso-occlusive coils described above (*see, e.g.,* U.S. Patent Nos. 6,723,112; 6,663,607; 6,602,269; 6,544,163; 6,287,318; 6,280,457 and 5,749,894).

Depicted in the Figures are exemplary embodiments of the present invention. Although the optional inner member is depicted as a coil, it will be appreciated that this is for purposes of illustration only and that the inner member can be of other shapes, for example different shaped coils, stents, mandrels, wires, filters or the like.

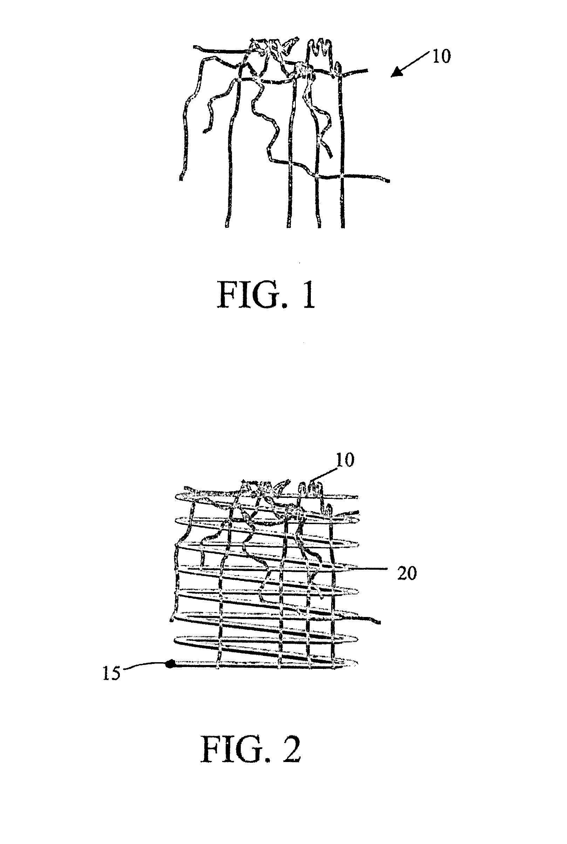

FIG. 1 depicts an exemplary embodiment of the inventive vaso-occlusive devices described herein. The device as a whole is generally designated (10) and is shown in a three-dimensional strand configuration.

FIG. 2 shows the exemplary embodiment of FIG. 1 in combination with GDC-type vaso-occlusive coil (20). As shown in FIG. 2, the strands of the non-degradable device may wind in and out of the turns making up the three-dimensional coil (*e.g.,* one or more of the strands passes through the lumen of the coil. Alternatively, the non-degradable device may be placed over the coil such that the strands are all on the exterior surface of the coil. Further, the non-degradable device (10) can be permanently or temporarily attached in one or more locations to the inner member by any suitable attachment mechanism. Also shown is detachment junction (15) positioned on the proximal end of the coil (20).

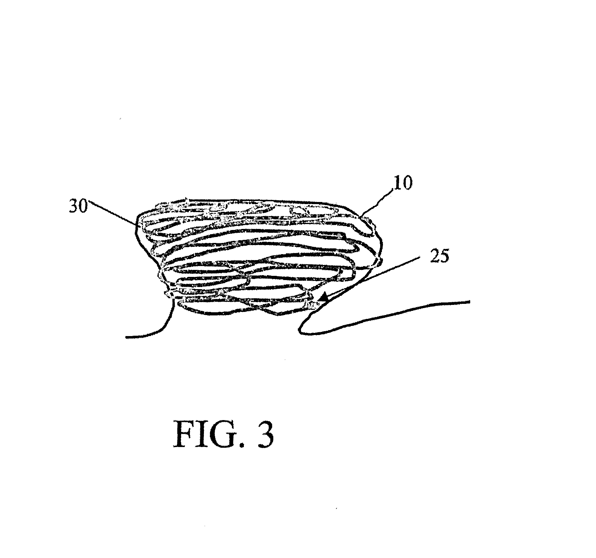

FIG. 3 shows an exemplary non-biodegradable device described herein, as deployed within an aneurysm (30). The device (10) assumes a three-dimensional configuration that substantially fills the aneurysm. Also shown in a small hook (25) at one end (*e.g.,* proximal end) of the device, which may aid in attachment to the wall of aneurysm. As shown, the inner member (coil) can be removed after the non-biodegradable member (30) has been deployed into the aneurysm. Optionally, additional (*e.g*., bioactive components) may be inserted into the aneurysm, for example into the spaces surrounding the filament.

The non-degradable devices described herein may assume a variety of structures including, but not limited to, braids, coil, stents (*e.g*., self-expanding stents) and combinations of these. Preferably, the devices are deployed in a primary linear configuration and assume a three-dimensional configuration upon deployment into the target vessel. For example, the devices may form a coil configuration or may have a substantially random space-filling relaxed configuration (FIG. 3) upon deployment.

Like the non-degradable component, the optional inner member may assume a variety of structure and be comprised of a variety of materials. Thus, although depicted in the Figures as a coil (*e.g*., platinum coil), the inner member may be of a variety of shapes or configuration including, but not limited to, braids, wires, knits, woven structures, tubes *(e.g.,* perforated or slotted tubes), injection-molded devices and the like. *See, e.g.,* U.S. Patent No. 6,533,801 and International Patent Publication WO 02/096273. Thus, the optional inner member may be made of a variety of materials, including but not limited to metals, polymers and combinations thereof.

In certain embodiments, the inner member is a braided structure comprising one or more metals or metal alloys, for example, Platinum Group metals, especially platinum, rhodium, palladium, rhenium, as well as tungsten, gold, silver, tantalum, stainless steel and alloys of these metals. Preferably, the inner member comprises a material that maintains its shape despite being subjected to high stress, for example, "super-elastic alloys" such as nickel/titanium alloys (48-58 atomic % nickel and optionally containing modest amounts of iron); copper/zinc alloys (38-42 weight % zinc); copper/zinc alloys containing 1-10 weight % of beryllium, silicon, tin, aluminum, or gallium; or nickel/aluminum alloys (36-38 atomic % aluminum). Particularly preferred are the alloys described in U. S. Pat. Nos. 3,174,851; 3,351,463; and 3,753,700. Especially preferred is the titanium/nickel alloy known as "nitinol." The inner member may also comprise a shape memory polymer such as those described in International Publication WO 03/51444.

In certain preferred embodiments, the inner member is a platinum coil. The inner member may also change shape upon release from the restraining member, for example change from a constrained linear form to a relaxed, three-dimensional configuration upon deployment.

As shown in FIG. 2, any of the devices described herein may further comprise a detachment junction (15), which is severable. The detachment junction (15) may be connected to a pusher element, such as a pusher wire. The detachment junction can be positioned anywhere on the device, for example at one or both ends of the optional inner member. In certain embodiments, the inner member may be removed after deployment, for example when the detachment junction is positioned at the proximal end of an inner member (FIG. 2).

The severable junction(s) may be detached in a variety of ways, for example using an electrolytically detachable assembly adapted to detach by imposition of a current; a mechanically detachable assembly adapted to detach by movement or pressure; a thermally detachable assembly adapted to detach by localized delivery of heat to the junction; a radiation detachable assembly adapted to detach by delivery of electromagnetic radiation to the junction or combinations thereof. Furthermore, the detachment mechanism may be hydraulic, for example the pusher wire may be cannulated, for example to allow for saline injection through the pusher wire to push off the coil.

The devices described herein may also comprise additional components, such as co-solvents, plasticizers, coalescing solvents, bioactive agents, antimicrobial agents, antithrombogenic agents (*e.g*., heparin), antibiotics, pigments, radiopacifiers and/or ion conductors which may be coated using any suitable method or may be incorporated into the element(s) during production. *See, e.g.,* co-owned U.S. Patent Application Serial No. 10/745,911, U.S. Patent No. 6,585,754 and WO 02/051460, incorporated by reference in their entireties herein. The bioactive materials can be coated onto the device (*e.g*., heparin) and/or can be placed in the vessel prior to, concurrently or after placement of one or more devices as described herein. For example, in embodiments in which the inner member is removed after deployment of the non-degradable device, one or more bioactive materials can be delivered to the vessel.

As noted elsewhere, the location of the device is preferably visible using fluoroscopy. A highly preferred method is to ensure that at least some of the elements (*e.g*., small pore non-degradable member and/or inner member) making up the device are provided with significant radio-visibility via the placement of a radio-opaque covering on these elements. A metallic coating of a metal having comparatively more visibility, during fluoroscopic use, than stainless steel is preferred. Such metals are well known but include gold and members of the Platinum Group described above.

One of more of the elements may also be secured to each other at one or more locations. For example, to the extent that various elements are thermoplastic, they may be melted or fused to other elements of the devices. Alternatively, they may be glued or otherwise fastened. Furthermore, the various elements may be secured to each other in one or more locations.

### METHODS OF USE

The devices described herein are often introduced into a selected site using the procedure outlined below. This procedure may be used in treating a variety of maladies. For instance in the treatment of an aneurysm, the aneurysm itself will be filled (partially or fully) with the compositions described herein.

Conventional catheter insertion and navigational techniques involving guidewires or flow-directed devices may be used to access the site with a catheter. The mechanism will be such as to be capable of being advanced entirely through the catheter to place vaso-occlusive device at the target site but yet with a sufficient portion of the distal end of the delivery mechanism protruding from the distal end of the catheter to enable detachment of the implantable vaso-occlusive device. For use in peripheral or neural surgeries, the delivery mechanism will normally be about 100-200 cm in length, more normally 130-180 cm in length. The diameter of the delivery mechanism is usually in the range of 0.25 to about 0.90 mm. Briefly, occlusive devices (and/or additional components) described herein are typically loaded into a carrier for introduction into the delivery catheter and introduced to the chosen site using the procedure outlined below. This procedure may be used in treating a variety of maladies. For instance, in treatment of an aneurysm, the aneurysm itself may be filled with the embolics (*e.g*. vaso-occlusive members and/or liquid embolics and bioactive materials) which cause formation of an emboli and, at some later time, is at least partially replaced by neovascularized collagenous material formed around the implanted vaso-occlusive devices.

A selected site is reached through the vascular system using a collection of specifically chosen catheters and/or guide wires. It is clear that should the site be in a remote site, e.g., in the brain, methods of reaching this site are somewhat limited. One widely accepted procedure is found in U.S. Patent into. 4,994,069 to Ritchart, et al. It utilizes a fine endovascular catheter such as is found in U.S. Patent No. 4,739,768, to Engelson. First of all, a large catheter is introduced through an entry site in the vasculature. Typically, this would be through a femoral artery in the groin. Other entry sites sometimes chosen are found in the neck and are in general well known by physicians who practice this type of medicine. Once the introducer is in place, a guiding catheter is then used to provide a safe passageway from the entry site to a region near the site to be treated. For instance, in treating a site in the human brain, a guiding catheter would be chosen which would extend from the entry site at the femoral artery, up through the large arteries extending to the heart, around the heart through the aortic arch, and downstream through one of the arteries extending from the upper side of the aorta. A guidewire and neurovascular catheter such as that described in the Engelson patent are then placed through the guiding catheter. Once the distal end of the catheter is positioned at the site, often by locating its distal end through the use of radiopaque marker material and fluoroscopy, the catheter is cleared. For instance, if a guidewire has been used to position the catheter, it is withdrawn from the catheter and then the assembly, for example including the vaso-occlusive device at the distal end, is advanced through the catheter.

Once the selected site has been reached, the vaso-occlusive device is extruded, for example by loading onto a pusher wire. Preferably, the vaso-occlusive device is loaded onto the pusher wire via a mechanically or electrolytically cleavable junction (*e.g*., a GDC-type junction that can be severed by application of heat, electrolysis, electrodynamic activation or other means). Additionally, the vaso-occlusive device can be designed to include multiple detachment points, as described in co-owned U.S. Patent No. 6,623,493 and 6,533,801 and International Patent publication WO 02/45596. They are held in place by gravity, shape, size, volume, magnetic field or combinations thereof.

As noted above, it will also be apparent that in certain embodiments, the inner member (20) is removeable after deployment into the vessel. For example, the device can be deployed into a vessel (aneurysm) as described herein. Once deployed, the non-degradable device (10) is detached from the inner member (20) while the inner member (20) remains attached to the pusher wire (25) and can be removed from the vessel.

It will also be apparent that the operator can remove or reposition (distally or proximally) the device. For instance, the operator may choose to insert a device as described herein, before detachment, move the pusher wire to place the device in the desired location.

Modifications of the procedure and vaso-occlusive devices described above, and the methods of using them in keeping with this invention will be apparent to those having skill in this mechanical and surgical art. These variations are intended to be within the scope of the claims that follow.

## Claims

1. A vaso-occlusive device comprising:
a plurality of strands comprising a porous non-biodegradable material having a pore size of less than about 20 microns; and
a vaso-occlusive inner member,
wherein the non-biodegradable material is attached to the inner member at one or more locations.

2. The device of claim 1, wherein the non-biodegradable material comprises a polymer.

3. The device of claim 2, wherein the polymer is selected from the group consisting of cellulose acetate, mixed esters cellulose, PTFE/polyester, acrylic copolymers and combinations thereof.

4. The device of claim 1, wherein the device has a linear primary configuration prior to deployment and a secondary three-dimensional configuration after deployment.

5. The device of claim 1, wherein the inner member comprises a metal.

6. The device of claim 5, wherein the metal is selected from the group consisting of nickel, titanium, platinum, gold, tungsten, iridium and alloys or combinations thereof.

7. The device of claim 6, wherein the metal is nitinol or platinum.

8. The device of claim 1, wherein the inner member comprises a coil.

9. The device of claim 1, wherein the inner member comprises a coil comprising a stainless steel or super-elastic metal alloy.

10. The device of claim 1, wherein the inner member further comprises a biodegradable material.

11. The device of claim 1, wherein the inner member further comprises a detachment junction.

12. The device of claim 11, wherein the detachment junction comprises an electrolytically detachable end adapted to detach from a pusher by imposition of a current on the pusher.

13. The device of claim 1, further comprising an additional component.

14. The device of claim 13, wherein the additional component is bioactive.
